# EUROPEAN PATENT APPLICATION

(11) **EP 3 639 794 A1**
(43) Date of publication of application: **22.04.2020**
(21) Application number: 18461617.5
(22) Date of filing: 18.10.2018
(51) Int. Cl.: A61F 2/80, A61F 2/68, A61F 2/76

(54) **AN ADJUSTABLE PROSTHETIC SOCKET AND A SUSPENSION SYSTEM**

(71) Applicant: VBIONIC Sp. z o.o., 60-612 Poznan (PL)
(72) Inventor: Rajewski, Bartosz, 60-612 Poznan (PL)
(74) Representative: Kancelaria Eupatent.pl Sp. z.o.o

(57) **Abstract**

An adjustable prosthetic socket characterized in that it comprises: a mesh comprising a plurality of mesh points (1) which are interconnected by at least one link (2), wherein each link (2) is configured to be individually adjusted to fit a shape and size of a stump; and an adapter (4), configured to be connected to a prosthesis, to which the mesh is fastened.

## Description

### TECHNICAL FIELD

The present invention relates to an adjustable prosthetic socket and a suspension system. In particular, the present invention relates to a prosthetic socket that can be adjusted to distribute a load evenly across an amputee's residual limb, regardless of volume and shape fluctuation.

### BACKGROUND OF THE INVENTION

A prosthetic socket is an element, which integrates the body with a prosthetic device. Each prosthetic socket is individually fitted and adjusted to the shape and volume of a patient's residual limb, depending on the level of amputation.

Traditionally, a prosthetic socket is manually made from thermoplastic polymers such as polypropylene. A patient has to visit a prosthetic clinic, at least twice - first to take measurements of a residual limb and next to try on a prosthetic socket.

The patient has to take functional tests so that the final modifications can be applied. It can take a lot of time to supply a device that does not always fit correctly so improvements have to be made.

A prosthetist has to provide a good contact surface and reproduce the shape of the stump accurately. The inner surface of the prosthetic socket has to correspond to the real dimensions of a user's stump. The aim is to eliminate the areas of excessive pressure, provide correct pressure where needed, protect sensitive areas and achieve a stable connection between the body and the prosthetic socket.

An improperly shaped socket contributes to premature fatigue, mechanical damage of the skin, discomfort and subsequently a patient's refusal to use said prosthesis. The outcome of the process depends on skills and experience of the prosthetist.

In traditional solutions, the way a prosthetic device is attached to the stump depends on proper contouring of the edges - it is important that the prosthetic socket stays on the stump while being exposed to external load. The prosthetic socket should not block movement in the joint while bending the limb. Sometimes, in case of short stumps, an additional mechanism (i.e., belts) is necessary to protect the device from slipping.

Apart from prosthetic sockets created from different types of molds, modular sockets are proposed (US20140135946A1), offering a regulation using straps and tensioners like BOA system (EP2805639A1).

The aim of the development of the present invention is an improved, adjustable prosthetic socket and suspension system.

### SUMMARY AND OBJECTS OF THE PRESENT INVENTION

The object of the invention is an adjustable prosthetic socket characterized in that it comprises: a mesh comprising a plurality of mesh points which are interconnected by at least one link, wherein each link is configured to be individually adjusted to fit a shape and size of a stump; and an adapter, configured to be connected to a prosthesis, to which the mesh is fastened.

Preferably, the operation of the mesh points results from pulling in or releasing the links between them thereby shortening or lengthening a distance between them.

Preferably, the operation of the mesh points results from an insertion or extension of a mechanical pin towards the stump.

Preferably, the plurality of mesh points are positioned on a protective layer.

Preferably, there are 160 or more mesh points per square meter.

Preferably, each of the plurality of mesh points is supplied with electrical power and comprises a sensor of its current state.

Preferably, each of the plurality of mesh points comprises an active segment configured to pull in and pull out the respective links of the mesh point as well as to extend or to shorten the mechanical pin.

Preferably, the socket further comprises control means between the active segment and a control unit in order to selectively control each active segment as well as to read its state.

Preferably, the socket further comprises an accelerometer.

Preferably, the socket further comprises a user's profile register configured to store the states of the active segments at different times.

Preferably, the user's profile register further stores data derived from the accelerometer at the different times.

Preferably, the controller is configured to set the active segments to a pre-programmed state in response to a control signal.

Another object of the invention is a method for controlling the adjustable prosthetic socket of any of previous claims, the method comprising the steps of: reading a current state of the active elements; reading time information; reading data from the accelerometer in order to establish an activity type of a user at the given time; creating a user's profile for a given time and activity type; and at a later time determining whether a current activity matches an activity having a stored profile and in case the current activity matches a known profile, executing the adjustment of the state of the active elements according to the determined profile.

There is also disclosed a computer program comprising program code means for performing all the steps of the computer-implemented method as described herein when said program is run on a computer, as well as a computer readable medium storing computer-executable instructions performing all the steps of the computer-implemented method as described herein when executed on a computer.

### BRIEF DESCRIPTION OF THE DRAWINGS

These and other objects of the invention presented herein, are accomplished by providing an adjustable prosthetic socket and suspension system. Further details and features of the present invention, its nature and various advantages will become more apparent from the following detailed description of the preferred embodiments shown in a drawing, in which:
Fig. 1 shows a three-dimensional concept of the invention;
Fig. 2 shows a possible way of construction of a mesh point along with the way of extending a movable pin section;
Fig. 3 shows a two-dimensional schematic diagram of the mesh points in which one of them generates perpendicular pressure on the stump by extending the moving part. Side view;
Fig. 4 shows a two-dimensional schematic diagram of the mesh points generating perpendicular pressure on the irregular part of the die by reducing the distance between the points. Side view;
Fig. 5 shows the two-dimensional mesh points that adapt to the irregularity of the stump between the points. View from above;
Fig. 6 shows different ways of adjusting the prosthetic socket;
Fig. 7 depicts a block diagram of the system according to the present invention; and
Fig. 8 presents a method according to the present invention.

### NOTATION AND NOMENCLATURE

Some portions of the detailed description which follows are presented in terms of data processing procedures, steps or other symbolic representations of operations on data bits that can be performed on computer memory. Therefore, a computer executes such logical steps thus requiring physical manipulations of physical quantities.

Usually these quantities take the form of electrical or magnetic signals capable of being stored, transferred, combined, compared, and otherwise manipulated in a computer system. For reasons of common usage, these signals are referred to as bits, packets, messages, values, elements, symbols, characters, terms, numbers, or the like.

Additionally, all of these and similar terms are to be associated with the appropriate physical quantities and are merely convenient labels applied to these quantities. Terms such as "processing" or "creating" or "transferring" or "executing" or "determining" or "detecting" or "obtaining" or "selecting" or "calculating" or "generating" or the like, refer to the action and processes of a computer system that manipulates and transforms data represented as physical (electronic) quantities within the computer's registers and memories into other data similarly represented as physical quantities within the memories or registers or other such information storage.

A computer-readable (storage) medium, such as referred to herein, typically may be non-transitory and/or comprise a non-transitory device. In this context, a non-transitory storage medium may include a device that may be tangible, meaning that the device has a concrete physical form, although the device may change its physical state. Thus, for example, non-transitory refers to a device remaining tangible despite a change in state.

As utilized herein, the term "example" means serving as a non-limiting example, instance, or illustration. As utilized herein, the terms "for example" and "e.g." introduce a list of one or more non-limiting examples, instances, or illustrations.

### DESCRIPTION OF EMBODIMENTS

The present invention enables control of volume and a shape of an entire prosthetic socket. In order to adjust the prosthetic socket to the residual limb's volume, a user has to adjust pressure at mesh points.

In case of a manual mode each mesh point has to be set separately. In an automatic mode all points can be adjusted at once by a socket electronic unit controlling, i.e., motors. The control unit can store saved settings as well as communicate with an external device such as a mobile phone to provide more sophisticated control over the prosthetic socket, i.e., different profiles changing over daytime automatically, on request or in some specific situations.

The prosthetic socket system provides a secure fit-once set, it keeps the position and pressure applied to the limb. Using the presented invention, a patient avoids unwanted relative motion between a residual limb and a prosthesis. Configuration settings can be provided both manually and automatically.

The user is able to control the pressure applied to the residual limb allowing the socket to stay in place firmly throughout any period of time. There is no need to take clothes or the whole prosthesis off. The openwork structure is beneficial for the residual limb - it deals with excessive heat and reduces skin irritations. A soft layer of material, which creates a protection against rubbing, preserves skin from strain, shear and harm, can be applied between the socket and the residual limb.

Maintaining a proper volumetric socket fit is challenging and often problematic due to the tendencies of a residual limb to change in volume and of the soft tissue to displace under load. As the stump changes through the day, the presented invention enables the user to adjust it (loosen or tighten the construction) as often as needed, all by themselves.

There is a possibility of releasing the stress while, i.e., sitting in the office, resting, not using the limb or when the stump swells, increasing its volume because of high temperatures. A patient can tighten the socket when going to a gym or carrying something heavy. The stump also changes its volume, shape and length when a person grows (especially children), gains or loses weight; a traditional socket requires a visit to a clinic to make corrections or requires making a completely new one. The present invention allows making changes individually, without prosthetist's help, without exchanging the socket and any additional costs.

The traditional socket is a rigid container surrounding the residual limb customized for each patient. An amputee inserts the stump in the rigid socket that limits the sensory experience in the body, which most patients describe as an inconvenience. In the present invention, sensing is maintained through a laced construction. The socket is compatible with the existing wrist connections, which means that any prosthetic device can be attached to it.

Contrary to the traditional method, there is no need to measure the residual limb, make a mold that corresponds to the residual limb or make the rigid support for it. As the patient does not visit a clinic, but purchases prosthetic socket remotely (online), the time of delivery is shortened, which means saving time and money. The socket is available in several universal sizes depending on patient's age, a length of the residual limb and a part of the body.

The presented socket system comprises a highly dense mesh of points that can be adjusted and that can produce tightening force towards the stump.

All the accompanying drawings are only explanatory and serve as an outline of the invention. The size, the arrangement (the distance and configuration) and the number of elements included in the mesh may vary.

For the purposes of the description of the drawings, a number was assigned to indicate a specific element of the invention. If there is more than one element of a kind, indexes in a form of lowercase letters, e.g., 1c or 2a, are used. This kind of identification allows for relating to a specific element precisely. The upper left-hand corner letters in the pictures (e.g., A, B) describe the use of the invention and indicate two phases of a particular situation.

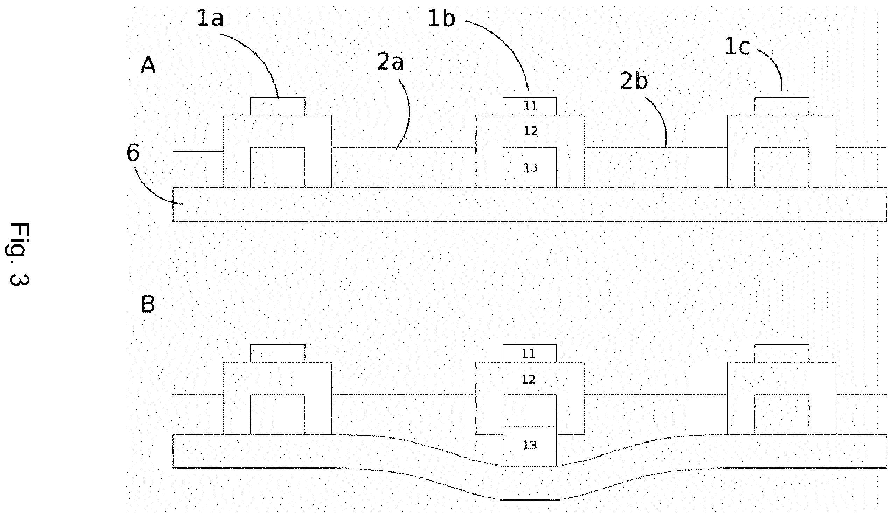

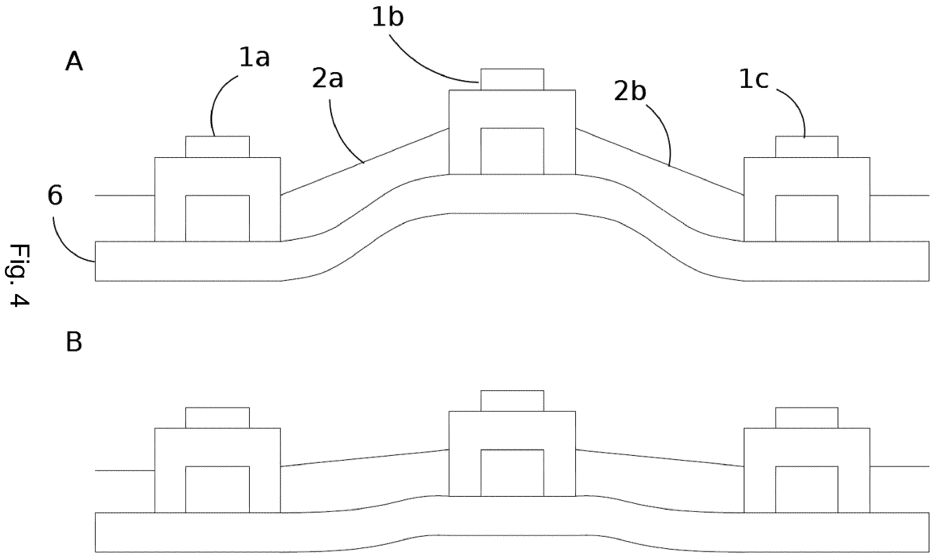

The term *mesh* is understood here as a cluster of interconnected points that can be adjusted in a way to enable the pressure to be generated on the stump. The way such pressure can be generated is presented in a later part of the description. In Fig. 3 and Fig. 4 hatching was skipped due to schematic character of the drawings, showing only the function of the presented element, not a detailed construction.

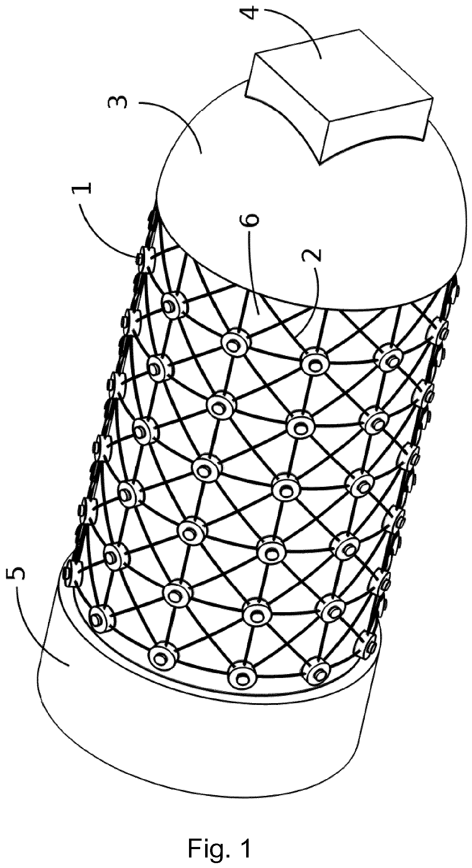

The notion of implementation of a prosthetic socket is shown in Fig. 1. Core components here are dense mesh points 1. A term the dense mesh means for example 160 or more mesh points 1 per square meter. The more points the better adjustment possibilities at an expense of weight and power consumption of the system.

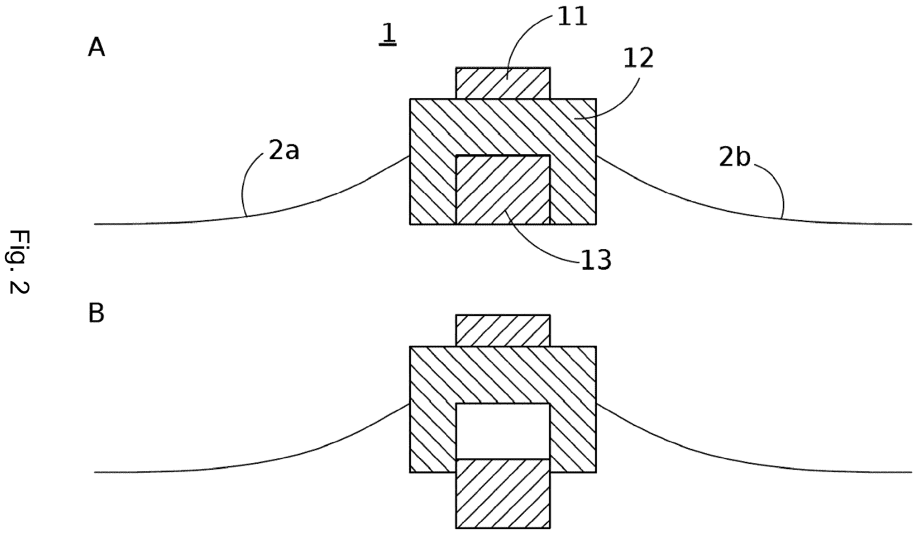

A detailed structure of said mesh points 1, is shown in FigG. 2. The points are interconnected with links 2 made of an inelastic material such as a polyethylene fiber or an aramid fiber.

The entire mesh of points is placed on a soft layer of material 6. The layer 6 should be thin and susceptible to forming, such as a fabric or foam made from natural materials such as cotton or artificial materials such as polyester. Its thickness is preferably equal to at least 6mm. Alternatively, a set of stacked layers of materials may be used. In general, the layer 6 is susceptible to changes of dimensions and adaptation to the user under the effect of functioning mesh points.

The layer 6 protects patient's skin from harm. A panel 5 contains a control system, a data storage, a power system and other elements improving the operation of said mesh points 1. The panel 5 may also comprise an accelerometer configured to detect physical activity in the automatic mode of the present invention.

An adapter 4, being a compatible connection with commercially available prostheses, is used to attach the prosthetic socket to a prosthesis properly. To ensure an optimal connection stability, a surface 3 may be used, which is a kind of a base. The surface 3 may have a shape of a dome but in general the shape shall match a shape of a stump and should facilitate mounting of the adapter 4.

The schematic diagram of the mesh point 1 is presented in Fig. 2. The element that may be placed in the mesh point is a sensor 11, able to read the position of links 2a and 2b as well as of an extending pin 13, and next to send this information to the panel 5 or directly to another device, e.g., a mobile phone.

Reading of the position parameters of the links 2a and 2b as well as of the extending pin 13, may be implemented using a device measuring angular rotation of a driving element such as an electric engine effector. Preferably, it is an encoder positioned at the end of an effector, which unambiguously determines rolling or unrolling of a link as well as the extension of the extending pin 13.

Determined position may be a difference between an initial positioning or a user-defined positioning of the links and a final positioning of the links. The initial positioning may be a factory-default setting.

By means of this information, operation of the mesh points 1 is controlled. An active segment 12 is responsible for pulling in the links 2a and 2b as well as is responsible for the extending pin 13. The active segment 12 may be a motor, a screw mechanism, a rope mechanism, an electromagnet, or a combination thereof or any other mechanism generating perpendicular force on the stump or between the mesh points 1.

In case of an electrical operation, the power supply and control may be effected in one of two ways: according to a first option, each mesh point 1 may be supplied with power and controlled by wires embedded in the layer 6 (or between its sublayers; according to a second option, each mesh point 1 has its own power source (such as a battery) and receives control signals wirelessly over a communication protocol such as Bluetooth, ZigBee or the like.

The extending pin 13 has a direct contact with the soft material layer 6 and, as already mentioned, it can be extended by means of the active segment 12. The schematic diagram of how such an action takes place, presents the transition from phase A, in which the extending pin is inserted, to phase B, in which it is extended. In a subsequent part of the description, the functions of the mesh point 1, i.e., the active segment 12, the sensor and the extending pin 13, are described as functions of the point, without specifying which element is responsible for it.

The operation mode of the socket is shown in Fig. 3. A point 1b extends the extending pin 13 that generates the intended perpendicular pressure on the soft material layer 6, and subsequently on the stump.

The pressure generation and the accompanying effects can be assessed from the transition from phase A to phase B, in which the mesh is bent, and in this way the pressure increases at this point. The level of extension can be set manually, electronically or automatically. Force generation can be supported by an operation of the links 2a and 2b, which form a connection between the points 1a, 1b and 1c. Shortening the distance between the point 1b and the adjacent points 1a or 1c, causes a greater stress between these points and results in generating the perpendicular pressure on the stump of a higher value.

The pressure generation can also take place only by means of the links 2a and 2b indicated in Fig. 4. In case of an irregular shape of the stump, e.g., a skin fold, greater or lesser pressure can be caused on the point. Passing from phase A to phase B, the links 2a and 2b are pulled in by the points 1a, 1b and 1c or a combination thereof, resulting in a lower bulge of the soft material 6, which reflects the irregularities of the stump.

In contrast, the transition from phase B to phase A is accompanied by a release of the links 2a and 2b, resulting in lower point pressure of the element 1b on the bulge of the soft material 6. Therefore, the previously distorted soft material 6, and the stump below it, can return to their natural position (in which there is no external pressure) or the distortion may change to a different position and subsequent desired point pressure.

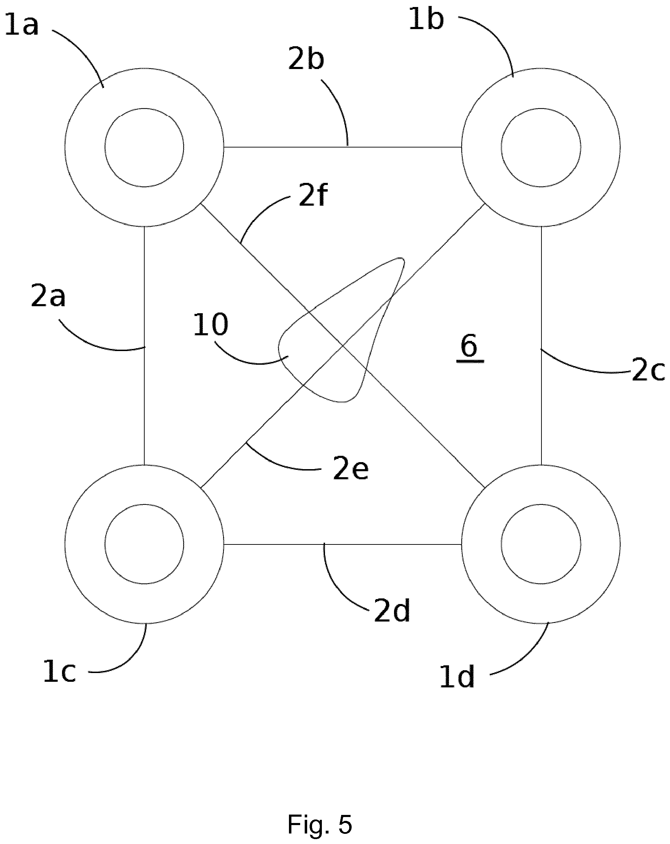

The soft material layer 6 adjustment options for irregular shapes placed directly under the points 1b are shown in Fig. 3 and Fig. 4. Fig. 5 illustrates a case, in which the bulge 10, being the reflection of the irregularities of the stump, placed between the points 1a, 1b, 1c and 1d, requires an adjustment. In this case, the operating principle of the invention is slightly different. Four sample adjustment cases (a) - (d) are presented below:
a) To reduce the perpendicular pressure on the bulge 10 caused by the links 2f and 2e, the links 2f and 2e, and optionally 2a, 2b, 2c, 2d, are released;
b) To reduce the perpendicular pressure, but at the same time to increase adhesion (i.e. minimizing the distance between the bulge 10 and the soft material 6 over the whole surface) to the bulge 10, the links 2f and 2e are released while the links 2a, 2b, 2c, 2d are pulled in. This may bring the points 1a, 1b, 1c and 1d closer to each other;
c) To increase the perpendicular pressure on the bulge 10, the links 2f and 2e are pulled in and optionally the links 2a, 2b, 2c, 2d are pulled in;
d) To increase the perpendicular pressure with decreasing the adherence to the bulge 10, the links 2f and 2e are pulled in and the links 2a, 2b, 2c and 2d are released.

The adjustment possibilities are optional, each of the links 2a, 2b, 2c, 2d, 2e, and 2f, can be individually pulled in or released, which allows changing the position of the mesh points 1a, 1b, 1c, 1d. Preferably, the links 2a, 2b, 2c, 2d, 2e, 2f may bend but may not stretch i.e. the material used has a high Young's modulus (an idealized rigid body would have an infinite Young's modulus). This, results in a better fit to the irregular shape of the stump and also to change in shape or size of the stump during an hour, a day, or any other time period.

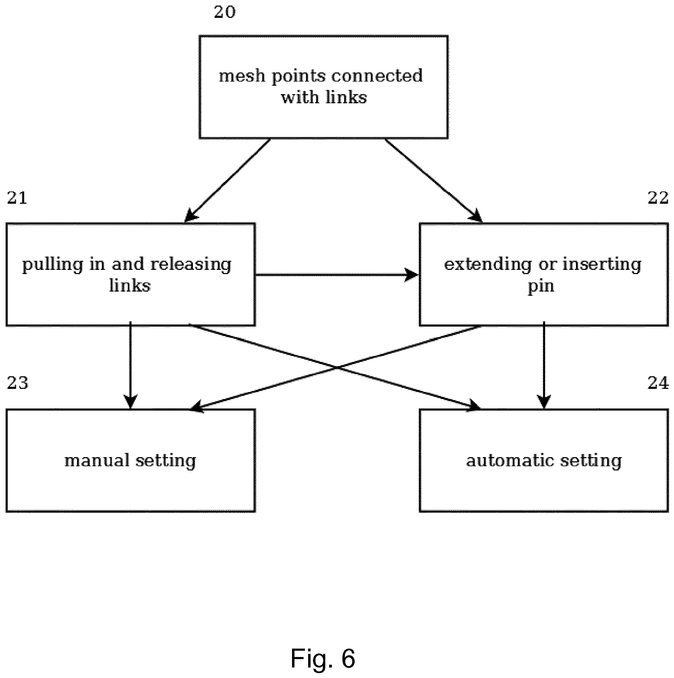

The ways, in which the mesh points 1 can be adjusted to the stump are shown in Fig. 6. The subject matter of the invention is based on a mesh of points connected with links 20. In this case the points are interconnected with the links, the adjustment to the stump can be effected by pulling in and releasing the links 23 and/or extending a mechanical pin. All adjusting operations can be performed manually or automatically. The invention may comprise any of the following options:
- 20, 21, 23 (i.e., a mesh of points whose operation is based on manual pulling in or releasing the links between them),
- 20, 21, 22, 23,
- 20, 21, 24,
- 20, 22, 23,
- 20, 22, 24,

In the case of points interconnected with the links 20, it is allowed to adjust the prosthetic socket by means of simultaneous pulling in/releasing the links 23 and extending the mechanical pin 22.

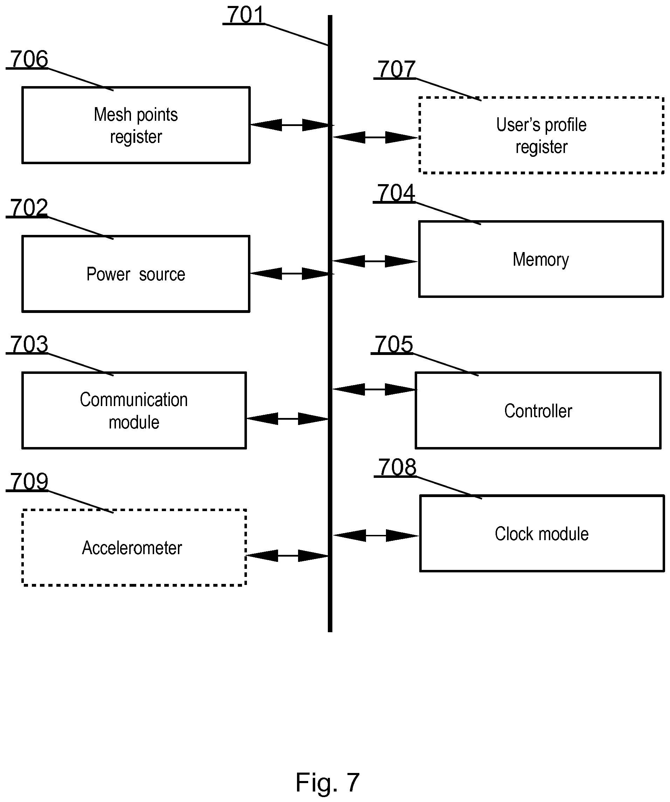

Fig. 7 depicts a block diagram of the system according to the present invention. The system may be realized using dedicated components or custom made FPGA or ASIC circuits. The system comprises a data bus 701 communicatively coupled to a memory 704. Additionally, other components of the system are communicatively coupled to the system bus 701 so that they may be managed by a controller 705.

The memory 704 may store computer program or programs executed by the controller 705 in order to execute steps of the method according to the present invention. Further, the memory 704 may store user's configuration as well as temporary data.

A power source module 702 will typically be a battery, preferably a rechargeable battery. This module may optionally power the active elements 12 via suitable wired connections as explained previously.

A communication module 703 may be present, which supports two optional purposes. The first is to communicate with the system for example from a smartphone. The other purpose is an optional communication with said active elements 12.

A mesh points register 706 may be present as a database of mesh points, their configuration and addressing options such that each mesh point may be unambiguously identified and communicated with. Such register will be present in an automatic version of the present invention.

A clock module 708 may be used to apply appropriate user profile as specified times wherein user's profile register 707 is configured to store said states of said active segments 12 recorded at different times and preferably with data from an accelerometer 709, which may be used to detect a type of activity of a user at different times e.g. during a sleep, a nap, a stroll, fa sast walk etc. Such activities may require different settings of said active elements.

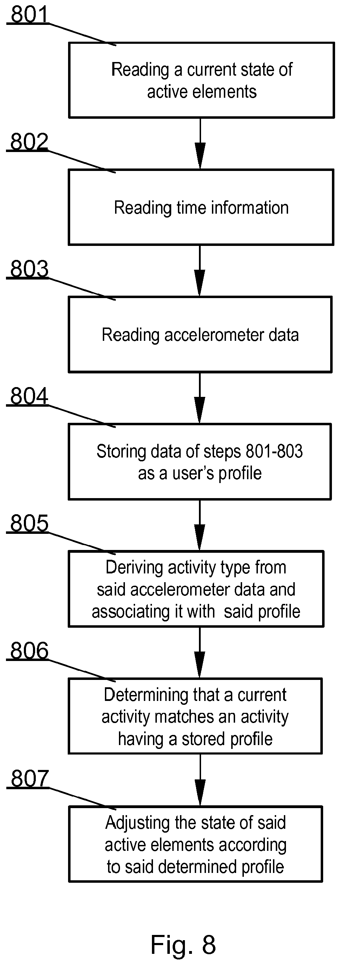

Fig. 8 presents a method according to the present invention. The aim of the method is to automatically set the active elements 12 of the mesh points 1. This in turn leads to a more comfortable use off the prosthesis for the user.

The method starts in step 801 from reading a current state of the active elements 12. This may be initiated after the user has provided an initial setting of the mesh points 1. Subsequently, in step 802, time information may be read, such as date and particular time of a day.

Next, in step 803, an accelerometer 709 data may be read in order to preferably establish an activity type of the user at given time.

The data, obtained in steps 801 - 803 create a user's profile for a given time and activity type 805. The profile information is stored in step 804 and supplemented with said activity type detected.

Next in step 806, the method determines whether a current activity matches an activity having a stored profile. The activity may be identified by a person's identifier, current date and time as well as optionally activity type which are all monitored in the background.

In case the current activity matches a known profile, then in step 807 adjustment of the state of said active elements according to said determined profile is executed.

Otherwise, when a new type of activity has been detected, a new profile may be associated and stored as associated with this new activity type.

An additional use-case, of the system according to the present invention, is putting the prosthesis on and off. During the putting on, the system may receive a signal from a user, that an initial position (per user's profile) shall be applied. Such signal may be provided via a physical button or wirelessly via a smartphone for example using the communication module 703. In other words, the controller 705 is configured to set the active segments 12 to a pre-programmed state in response to a control signal.

After such initial setting, said monitoring according to Fig. 8 may take place.

Similarly, when putting the prosthesis off, a user may inform the system in a similar manner so that the active elements will loosen their grip appropriately in order to facilitate removal.

At least parts of the methods according to the invention may be computer implemented. Accordingly, the present invention may take the form of an entirely hardware embodiment, an entirely software embodiment (including firmware, resident software, micro-code, etc.) or an embodiment combining software and hardware aspects that may all generally be referred to herein as a "circuit", "module" or "system".

Furthermore, the present invention may take the form of a computer program product embodied in any tangible medium of expression having computer usable program code embodied in the medium.

It can be easily recognized, by one skilled in the art, that the aforementioned method for operating an adjustable prosthetic socket may be performed and/or controlled by one or more computer programs. Such computer programs are typically executed by utilizing the computing resources in a computing device. Applications are stored on a non-transitory medium. An example of a non-transitory medium is a non-volatile memory, for example a flash memory while an example of a volatile memory is RAM. The computer instructions are executed by a processor. These memories are exemplary recording media for storing computer programs comprising computer-executable instructions performing all the steps of the computer-implemented method according the technical concept presented herein.

While the invention presented herein has been depicted, described, and has been defined with reference to particular preferred embodiments, such references and examples of implementation in the foregoing specification do not imply any limitation on the invention. It will, however, be evident that various modifications and changes may be made thereto without departing from the broader scope of the technical concept. The presented preferred embodiments are exemplary only, and are not exhaustive of the scope of the technical concept presented herein.

Accordingly, the scope of protection is not limited to the preferred embodiments described in the specification, but is only limited by the claims that follow.

## Claims

1. An adjustable prosthetic socket **characterized in that** it comprises:
- a mesh comprising a plurality of mesh points (1) which are interconnected by at least one link (2), wherein each link (2) is configured to be individually adjusted to fit a shape and size of a stump; and
- an adapter (4), configured to be connected to a prosthesis, to which the mesh is fastened.

2. The adjustable prosthetic socket according to claim 1, wherein the operation of the mesh points results from pulling in or releasing the links between them thereby shortening or lengthening a distance between them.

3. The adjustable prosthetic socket according to claim 1, wherein the operation of the mesh points (1) results from an insertion or extension of a mechanical pin (13) towards the stump.

4. The adjustable prosthetic socket according to claim 1, wherein the plurality of mesh points (1) are positioned on a protective layer (6).

5. The adjustable prosthetic socket according to claim 1, wherein there are 160 or more mesh points (1) per square meter.

6. The adjustable prosthetic socket according to claim 1, wherein each of the plurality of mesh points (1) is supplied with electrical power (702) and comprises a sensor of its current state.

7. The adjustable prosthetic socket according to claim 6, wherein each of the plurality of mesh points (1) comprises an active segment (12) configured to pull in and pull out the respective links (2) of the mesh point (1) as well as to extend or to shorten the mechanical pin (13).

8. The adjustable prosthetic socket according to claim 7, wherein it further comprises control means between the active segment (12) and a control unit (405) in order to selectively control each active segment (12) as well as to read its state.

9. The adjustable prosthetic socket according to claim 6, further comprising an accelerometer (709).

10. The adjustable prosthetic socket according to claim 8, further comprising a user's profile register (707) configured to store the states of the active segments (12) at different times.

11. The adjustable prosthetic socket according to claims 8 and 9, wherein the user's profile register (707) further stores data derived from the accelerometer (709) at the different times.

12. The adjustable prosthetic socket according to claims 8 and 9, wherein the controller (705) is configured to set the active segments (12) to a pre-programmed state in response to a control signal.

13. A method for controlling the adjustable prosthetic socket of any of previous claims, the method comprising the steps of:
- reading (811) a current state of the active elements (12);
- reading (802) time information;
- reading (803) data from the accelerometer (709) in order to establish an activity type of a user at the given time;
- creating (805) a user's profile for a given time and activity type; and
- at a later time (806) determining whether a current activity matches an activity having a stored profile and in case the current activity matches a known profile, executing (807) the adjustment of the state of the active elements (12) according to the determined profile.

14. A computer program comprising program code means for performing all the steps of the computer-implemented method according to claim 13 when said program is run on a computer.

15. A computer readable medium storing computer-executable instructions performing all the steps of the computer-implemented method according to claim 13 when executed on a computer.
